# EUROPEAN PATENT SPECIFICATION

(11) **EP 4 124 350 B1**
(45) Date of publication and mention of the grant of the patent: **11.12.2024**
(21) Application number: 21188194.1
(22) Date of filing: 28.07.2021
(51) Int. Cl.: B64D 11/00, B60Q 3/41, A61L 2/10

(54) **SYSTEM AND METHOD FOR PROVIDING AND MONITORING UV ILLUMINATION IN AN INTERIOR OF AN AIRCRAFT**
SYSTEM UND VERFAHREN ZUR BEREITSTELLUNG UND ÜBERWACHUNG DER UV-BELEUCHTUNG IN EINEM INNENRAUM EINES FLUGZEUGES
SYSTÈME ET PROCÉDÉ DE FOURNITURE ET DE SURVEILLANCE D'ÉCLAIRAGE UV DANS L'INTÉRIEUR D'UN AÉRONEF

(43) Date of publication of application: 01.02.2023
(73) Proprietor: Goodrich Lighting Systems GmbH & Co. KG, 59557 Lippstadt (DE)
(72) Inventor: HESSLING- VON HEIMENDAHL, Andre, 56073 Koblenz (DE); JOHANNESSEN, Eric, Holbrook, NY, 11741 (US)
(74) Representative: Schmitt-Nilson Schraud Waibel Wohlfrom Patentanwälte Partnerschaft mbB

(56) References cited:
- WO-A1-2021/195003
- CN-A- 111 955 811
- DE-T2- 69 309 247
- US-A1- 2018 021 465
- US-B2- 10 918 749
- US-B2- 8 084 752
- ANONYMOUS: "Light Conversion using Perovskite Quantum Dots - Fluxim", 20 April 2020 (2020-04-20), pages 1 - 7, XP055877541, Retrieved from the Internet <URL:https://web.archive.org/web/20200420175501/https://www.fluxim.com/light-conversion-perovskite-quantum-dots> [retrieved on 20220111]

## Description

The present invention is in the field of aircraft equipment. The present invention is in particular in the field of aircraft disinfection, more particularly in the field of aircraft disinfection using ultraviolet light. In the following, ultraviolet light will be referred to as "UV light".

For reducing the risk of distributing infectious diseases in an aircraft, it is desirable to regularly disinfect surfaces within the aircraft, which are routinely contacted by passengers and/or aircraft personnel. UV light may be used as a germicidal illumination for disinfecting the surfaces within an aircraft by illumination.

WO 2021/195003 A1 disclose systems and methods for disinfecting a populated venue/space in real-time by autonomously employing directed disinfecting light/radiation. One embodiment comprises an ultraviolet light source which may comprise coherent and incoherent light sources that disinfect surfaces of pathogens; a set of sensors collecting data correlating to characteristics of a plurality of objects and events in the space; and a controller that receives data from the set of sensors; identifies areas to be disinfected; identifies whether the ultraviolet light source has a clear line-of-sight path to irradiate the area; and sends control signals to the light source to control the application of disinfecting light to areas in the space.

US 8 084 752 B2 discloses a keyboard and touchpad or mouse ultraviolet treatment system with optical sensor and inclined slide is described. A proximity sensor, microcontroller firmware, and motorized mechanism allow activation of the sanitization cycle by personnel with contaminated hands without risk of additional spreading of pathogens to other personnel and patients. The inclined slide provides stability when operated on a desk, and the microcontroller and optical sensor determine the proper exposure time to compensate for lamp aging and variations in lamp output.

It would be beneficial to provide a system and a method which provide an effective framework for disinfection via UV light in an aircraft.

According to an exemplary embodiment of the invention, a system for providing and monitoring germicidal UV illumination in an interior of an aircraft comprises at least one switchable UV light source; a light detector, which is responsive to visible light and UV light and which is configured for providing sensor outputs regarding detected light; and a controller for monitoring an operational status of the at least one switchable UV light source. The controller is configured for determining the operational status of the at least one switchable UV light source by comparing a first sensor output, provided by the light detector when the at least one switchable UV light source is activated, with a second sensor output, provided by the light detector when the at least one switchable UV light source is deactivated.

According to an exemplary embodiment of the invention, a method for providing and monitoring germicidal UV illumination in an interior of an aircraft comprises detecting light, which may include visible light and UV light, within the interior of the aircraft, while at least one UV light source within the interior of the aircraft is deactivated, with a light detector providing a first sensor output; activating the at least one UV light source; detecting light, which may include visible light and UV light within the interior of the aircraft, while the at least one UV light source provided within the interior of the aircraft is activated, with the light detector providing a second sensor output; and monitoring an operational status of the at least one switchable UV light source, wherein monitoring an operational status of the at least one switchable UV light source includes comparing the first sensor output with the second sensor output.

Systems and methods providing and monitoring UV illumination in an interior of an aircraft according to exemplary embodiments of the invention allow for an efficient and reliable disinfection of surfaces within an aircraft using UV light. They may further allow for monitoring and documenting the emission of UV light. This may allow for proving that sufficient disinfection has been carried out.

Exemplary embodiments of the invention may allow for carrying out disinfection within an aircraft automatically and for adjusting the disinfection process to a potential aging and/or deterioration of the at least one UV light source, which is employed for generating the UV light.

In an embodiment, the at least one UV light source is configured for emitting UV electromagnetic radiation having a wavelength of less than 400 nm, in particular a wavelength in the range of between 150 nm and 300 nm, further in particular a wavelength in the range of between 200 nm and 270 nm. Electromagnetic radiation having a wavelength in these ranges has been found as highly efficient for disinfection.

In an embodiment, the light detector includes a photo sensor, which is sensitive to visible light, and a wavelength converting coating. The wavelength converting coating emits visible light when being excited by UV light. Such a configuration may allow for using a comparatively inexpensive photo diode, which is sensitive to visible light, as a photo sensor. Further, as the light emitted by the wavelength converting coating, when it is excited by UV light, is visible to the human eye, humans may recognized the presence of invisible UV light directly, i.e. without the assistance of a photo sensor, by sensing the visible light emitted by the wavelength converting coating.

In an embodiment, the wavelength converting coating comprises a light transmissive adhesive, in particular a translucent adhesive, which is applied to the photo sensor, and a wavelength converting substance, which emits visible light when it is excited by UV light. The light transmissive adhesive may be a silica based material, for example a silicone or a sodium silicate.

The wavelength converting substance may be applied to an outer surface of the light transmissive adhesive, in particular to an outer surface opposite to the photo sensor. Such a configuration may allow for illuminating the wavelength converting substance with UV light, without the UV light passing through the adhesive. If the UV light does not have to pass through the adhesive, there is no risk that the UV light is attenuated or even blocked by the adhesive. Further, the adhesive may be embodied to be light transmissive only to visible light, but not to UV light. This provides more options for selecting an appropriate adhesive.

In an embodiment, the light transmissive adhesive may include a thin quartz window that encapsulates the wavelength converting substance underneath.

In an embodiment, the wavelength converting substance is applied only to a portion of the outer surface of the light transmissive adhesive. This may allow visible light to pass through the portions of the light transmissive adhesive, to which no wavelength converting substance is applied, in a particularly unimpeded manner. Such a configuration may allow the photo sensor to have high sensitivity not only to light, which is emitted by the wavelength converting substance when its is excited by UV light, but also to visible light incident on the light detector.

Additionally or alternatively, the wavelength converting coating may be applied to only a portion of a light detecting surface. This may allow visible light to illuminate surface portions of the photo sensor, which are not covered by the wavelength converting coating, in a particularly unimpeded manner.

In an embodiment, the photo sensor is configured for providing spectral information about the light detected by the photo sensor. The photo sensor may in particular provide information about the intensity of the detected light as a function of the wavelength of the detected light. Providing spectral information about the light detected by the photo sensor may be a particularly effective way of distinguishing between UV light and visible light illuminating the light detector. As a result, the operation of the UV light source may be monitored very effectively and highly reliably.

In an embodiment, the photo sensor comprises a plurality of detection channels, wherein the plurality of detection channels have different sensitivities to different ranges of electromagnetic radiation. As a result, the photo sensor may be capable to provide separate intensity information for each of a plurality of ranges of electromagnetic radiation.

In an embodiment, the photo sensor is a multi-color photo sensor, wherein the plurality of detection channels have different sensitivities to different ranges of electromagnetic radiation in the range of visible light.

In an embodiment, the wavelength converting substance includes a phosphor material. The wavelength converting substance may in particular include at least one of Y₂O₃:Eu³⁺, Sr₂AL₆O₁₁:Eu²⁺, BaMgAl₁₀O₁₇:Eu²⁺, and LaPO₄:Ce³⁺,TB³⁺.

Y₂O₃:Eu³⁺, Sr₂AL₆O₁₁:Eu²⁺, BaMgAl₁₀O₁₇:Eu²⁺, and LaPO₄:Ce³⁺,TB³⁺ have been found as very suitable and efficient materials for converting UV light into visible light.

When excited by UV light, Y₂O₃:Eu³⁺ emits red light, in particular red light having wavelengths in the range of between 605 nm and 615 nm, in particular red light having wavelengths which are basically centered around 611 nm.

When excited by UV light, Sr₂AL₆O₁₁:Eu²⁺ emits blue light, in particular blue light having wavelengths in the range of between 425 nm and 475 nm, in particular blue light having wavelengths which are basically centered around 450 nm.

When excited by UV light, BaMgAl₁₀O₁₇:Eu²⁺ emits blue light, in particular blue light having wavelengths in the range of between 430 nm and 480 nm, in particular blue light having wavelengths which are basically centered around 455 nm.

When excited by UV light, LaPO₄:Ce³⁺,TB³⁺ emits green light, in particular green light having wavelengths in the range of between 520 nm and 570 nm, in particular green light having wavelengths which are basically centered around 545 nm.

In an embodiment, the wavelength converting substance includes quantum dots for converting UV light into visible light.

In an embodiment, the system is configured for and the method includes controlling the at least one switchable UV light source based on the monitoring of the operational status of the at least one switchable UV light source. Controlling the at least one switchable UV light source may in particular include adjusting an intensity of the UV light, emitted by the at least one switchable UV light source, and/or adjusting a period of time, for which the at least one switchable UV light source is activated. This may allow for adjusting the operation of the at least one switchable UV light source so that an efficient and reliable disinfection of the surfaces, which are illuminated with the UV light emitted by the UV light source, is ensured.

In an embodiment, the system is provided in a cockpit of an aircraft and it may be configured for illuminating at least one surface, which is usually touched by the pilots, with UV light for disinfection.

In an embodiment, the system is provided in a passenger cabin of an aircraft and it may be configured for illuminating at least one surface, which is usually touched by the passengers and/or cabin crew members, with UV light for disinfection. The system may, for example, be configured for illuminating the passenger seats, in particular the arm rests and/or the head rests of the passengers seats, and/or passenger service units (PSUs), which are arranged above the passenger seats, with UV light for disinfection.

In an embodiment, the system is provided in a lavatory and/or in a galley, provided within the passenger cabin of the aircraft, for disinfecting the lavatory and/or the galley, respectively. Multiple such systems may be provided in the lavatories and/ or galleys and/or seating portions of the passenger cabin.

In an embodiment, the system(s) for providing and monitoring UV illumination according to exemplary embodiments of the invention is/are activated only after the passengers and crew have left the aircraft after landing, and the system(s) is/are deactivated before new passengers start boarding the aircraft, so that no people are present within the aircraft when the system(s) is/are activated. Activating the system only when no passengers and/or crew members are present within the aircraft may prevent humans from being irradiated with UV light.

In an embodiment, system(s) provided in lavatories of the aircraft may be additionally activated during flight when the lavatories are not occupied and the doors of the lavatories are closed, so that no UV light can exit the lavatory. Systems provided in the lavatories may, for example, be activated at regular time intervals or after a predefined number of passengers have used the respective lavatory, in order to ensure hygienic conditions within the lavatories during the flight.

In an embodiment, controlling the at least one switchable UV light source includes adjusting the intensity of UV light emitted by the at least one switchable UV light source by controlling the operation of the UV light source. The intensity of UV light emitted by the at least one UV light source may, for example, be adjusted so that the intensity of the UV light is within a predefined range between a predefined minimum intensity and a predefined maximum intensity.

In an embodiment, controlling the at least one switchable UV light source includes adjusting a period of time, for which the at least one switchable UV light source is activated. The period of time, for which the at least one switchable UV light source is activated, may in particular be adjusted as a function of the detected intensity of UV light.

For example, the period of time, for which the at least one switchable UV light source is activated, may be extended when the detected UV intensity is low, and the period of time, for which the at least one switchable UV light source is activated, may be shortened when the detected UV intensity is high, in order to achieve a substantially constant disinfection effect, independent of potential changes of the intensity of the UV light. The intensity of the UV light may vary for example due to aging of the at least one UV light source, due to contamination of the UV light source, etc..

In an embodiment, the controller is configured for calculating an indication regarding a total amount of UV light, which is emitted by the at least one switchable UV light source over time, by accumulating the sensor outputs over time. The controller may further be configured for providing a confirmation signal when the indication exceeds a predefined threshold. The confirmation signal may include a visual and/or an acoustic signal.

Such a configuration may allow for indicating that an amount of UV light, which is considered as sufficient for reliably disinfecting at least one surface illuminated with the UV light, has been detected.

The confirmation signal may also be employed as a control signal, which causes the at least one switchable UV light source to be deactivated. This may allow for automatically deactivating the at least one switchable UV light source, after a sufficient amount of UV light has been emitted. In consequence, surfaces within the aircraft may be disinfected automatically without human intervention and without excessive use of UV light.

In an embodiment, the confirmation signal includes a signal which is transmitted to a board computer of the aircraft and/or a report / log signal, which allows for logging the operation of the at least one UV light source. Logging the operation of the at least one UV light source may allow for documenting that surfaces within the aircraft have been disinfected regularly and sufficiently.

Logging the operation of the at least one UV light source may further allow for monitoring the periods of operational time of the at least one UV light source needed for sufficient disinfection. An increase of the periods of operational time needed for sufficient disinfection may indicate an aging of the at least one UV light source. In consequence, an aging UV light source may be replaced in good time before it completely fails or becomes too weak for reliable disinfection.

Further exemplary embodiments of the invention are described below with respect to the accompanying drawings, wherein:
Figure 1 depicts an aircraft, in particular an air plane, in accordance with an exemplary embodiment of the invention in a schematic side view.
Figure 2 depicts a schematic view of an overhead passenger service unit (PSU).
Figure 3 depicts a schematic cut-open view of an aircraft in accordance with an exemplary embodiment of the invention, depicting a passenger cabin of the aircraft.
Figure 4 depicts a schematic view of a system for providing and monitoring UV illumination according to an exemplary embodiment of the invention.
Figure 5 shows a schematic cross-sectional view of a light detector, as it may be employed in a system according to an exemplary embodiment of the invention.
Figures 6A shows the light intensities, detected by the light detector, as a function of the wavelength, when the at least one UV light source is switched off.
Figures 6B shows the light intensities, detected by the light detector, as a function of the wavelength, when the at least one UV light source is switched on.

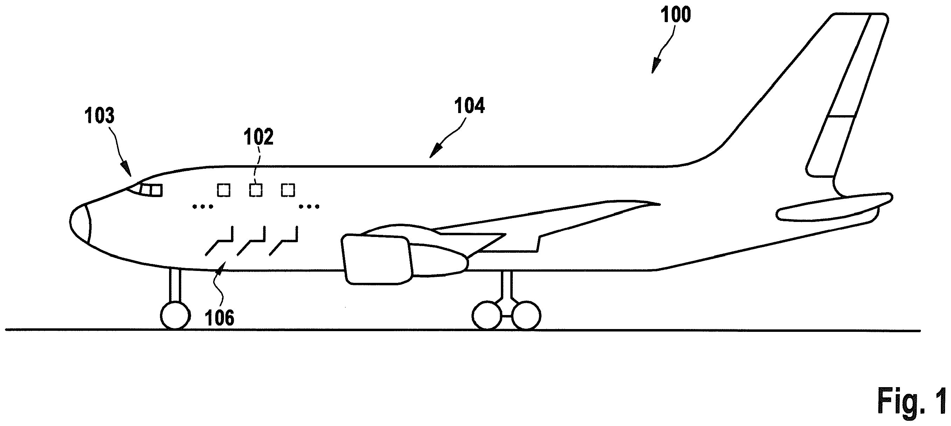

Figure 1 shows an aircraft 100, in particular an air plane, in accordance with an exemplary embodiment of the invention in a schematic side view. In the exemplary embodiment shown in Figure 1, the aircraft 100 is a large passenger air plane, comprising a cockpit 103 and a passenger cabin 104 housing a plurality of passenger seats 106. The aircraft 100 may be a commercial passenger air plane, a private air plane, or a military aircraft. It is also possible that the system and method according to exemplary embodiments of the invention are implemented in a rotor-craft, such as a helicopter.

Passenger service units (PSU) 102 are arranged above the passenger seats 106.

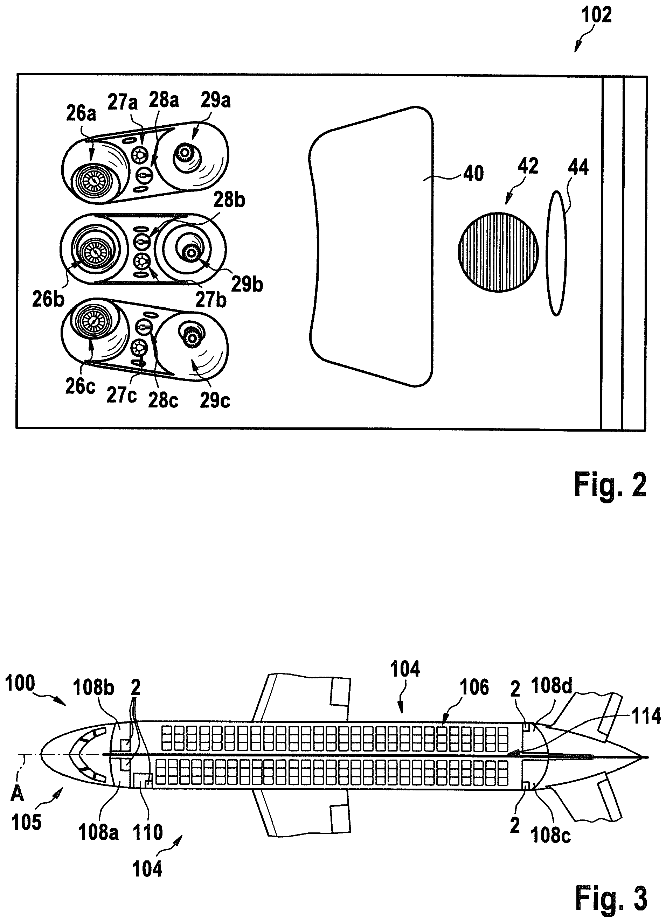

In an exemplary configuration, in which the aircraft 100 comprises six passenger seats 106 per row (cf. Figure 3, which will be discusses in more detail further below), each row of passenger seats 106 may have two passenger service units 102 associated therewith, one passenger service unit 102 assigned to the passenger seats 106 on the left side of a center aisle 114 and one passenger service unit 102 assigned to the passenger seats 106 on the right side of the center aisle 114.

Figure 2 depicts a schematic view of an overhead passenger service unit (PSU) 102, which is arranged above the passengers of a single passenger row, as it is seen from the side of a passenger sitting on a passenger seat 106 below the overhead passenger service unit 102.

On the side, which is shown to the left in Figure 2, the overhead passenger service unit 102 comprises a row of three adjustable reading lights 26a-26c, which are arranged next to each other.

Six electrical switches 27a-27c, 28a-28c are provided to the right side of the reading lights 26a-26c, a respective pair of two switches 27a-27c, 28a-28c next to each of the reading lights 26a-26c. One of the switches 27a-27c of each pair is configured for switching the adjacent reading light 26a-26c, and the second switch 28a-28c of each pair is configured for triggering a signal for calling cabin service personnel.

A row of three adjacent gaspers 29a-29c is provided next to the switches 27a-27c, 28a-28c.

Adjacent to the gaspers 29a-29c is a removable cover 40, which covers a cavity housing at least three oxygen masks (not shown). In the event of pressure loss within the cabin, the removable cover 40 will open, the oxygen masks will drop out of the cavity and each of the passengers, sitting below the overhead passenger service unit 102, may grasp one of the oxygen masks. The oxygen masks will be supplied with oxygen allowing the passengers to continue to breathe normally.

On the side opposite to the gaspers 29a-29c, a grid 42 is formed within overhead passenger service unit 102. A loudspeaker (not shown), which may be used for delivering acoustic announcements to the passengers, is arranged behind said grid 42.

Next to the grid 42, there is a display panel 44, which may be configured for selectively showing a plurality of visual signs (not shown), such as "non smoking" or "fasten you seat belt". The display panel 44 may be illuminated from behind, in order to deliver visual information to the passengers sitting below the overhead passenger service unit 102.

Figure 3 depicts a schematic cut-open view of an aircraft 100 in accordance with an exemplary embodiment of the invention, depicting a passenger cabin 104 of the aircraft 100, also referred to as aircraft passenger cabin 104 herein.

The aircraft passenger cabin 104 is equipped with a plurality of passenger seats 106. The passenger seats 106 are arranged next to each other forming a plurality of passenger seat rows. Each passenger seat row comprises two groups of passenger seats 106, respectively including three passenger seats 106. The two groups of passenger seats 106 are separated from each other by a center aisle 114, extending along a longitudinal axis A of the aircraft 1.

The aircraft passenger cabin 104 is further equipped with four lavatories 108a-108d. In the exemplary configuration depicted in Figure 3, lavatories 108a-108d are provided at four locations within the aircraft passenger cabin 104. A first lavatory 108a is located at the front portside end of the aircraft passenger cabin 104, a second lavatory 108b is located at the front starboard end of the aircraft passenger cabin 104, a third lavatory 108c is located at the rear portside end of the aircraft passenger cabin 104, and a fourth lavatory 108d is located at the rear starboard end of the aircraft passenger cabin 104. Additionally or alternatively, lavatories 108a-108d may be provided at other locations of the aircraft passenger cabin 104 as well.

The aircraft passenger cabin 104 is further equipped with a galley 110, in order to allow for preparing meals and drinks for the passengers.

At least one of the lavatories 108a-108d and the galley 110 is provided with a system 2 for providing and monitoring UV illumination according to an exemplary embodiment of the invention.

In the exemplary embodiment depicted in Figure 3, each lavatory 108a-108d and the galley 110 are provided with a system 2 for providing and monitoring UV illumination, respectively. However, exemplary embodiments of the invention also include aircraft 100 in which only one or any subset of the lavatories 108a-108d and the galley 110 are provided with a system 2 for providing and monitoring UV illumination.

Although not explicitly depicted in Figure 3, systems 2 for providing and monitoring UV illumination according to exemplary embodiments of the invention may also be provided next to the passenger service units 102 (cf. Figures 1 and 2) for disinfecting said passenger service units 102 by illuminating the passenger service units 102 with UV light.

Systems 2 for providing and monitoring UV illumination according to exemplary embodiments may also be provided within the passenger cabin 104 for illuminating and disinfecting the passenger seats 106 located under the passenger service units 102. The systems 2 may, for example, be integrated into the passenger service units 102 or arranged next to the passenger service units 102.

Systems 2 for providing and monitoring UV illumination according to exemplary embodiments of the invention may also be provided within the cockpit 103 of the aircraft 100 for disinfecting surfaces touched by the pilots.

As UV light may be harmful to humans, in particular to the human eye, the systems 2 for providing and monitoring UV illumination according to exemplary embodiments provided within the passenger cabin 104 and/or within the cockpit 103 of the aircraft 100 may be activated only after the passengers and crew have disembarked after the flight, so that no humans are present within the aircraft 100.

Systems 2 for providing and monitoring UV illumination according to exemplary embodiments located in the lavatories 108a-108d, however, may also be activated during flight, when the lavatories 108a-108d are not occupied and the doors of the lavatories 108a-108d are closed, so that no UV light can exit the lavatories 108a-108d. Systems 2 located within the lavatories 108a-108d may, for example, be activated in regular time intervals or after a predefined number of passengers have used the respective lavatory 108a-108d, in order to ensure hygienic conditions within the lavatories during the flight.

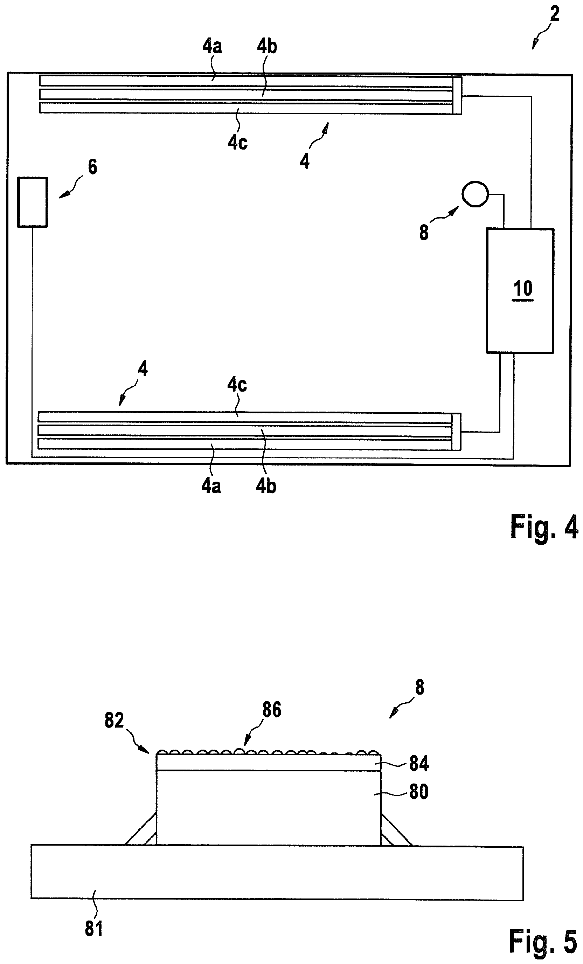

A schematic view of a system 2 for providing and monitoring UV illumination according to an exemplary embodiment of the invention is depicted in Figure 4.

The system 2 comprises at least one lighting device 4, which is configured for emitting visible light for illuminating the environment of the system 2, for example the lavatory 108a-108d or the galley 110. In the exemplary embodiment depicted in Figure 4, two lighting devices 4 are shown. Each lighting device 4 may comprises at least one LED acting as a light source. Each lighting device 4 may in particular comprise a plurality of light sources 4a-4c, which are configured for emitting light of different colors. Such a configuration may allow for adjusting the color of the light, emitted by the at least one lighting device 4, by varying the intensity of the light emitted by the different light sources 4a-4c. In the exemplary embodiment of Figure 4, each of the plurality of light sources 4a-4c comprises one or more LED(s).

The system 2 further comprises at least one switchable UV light source 6, which is configured for emitting UV light when activated. The UV light emitted by the at least one UV light source 6 may be directed to at least one surface for disinfecting said at least one surface, using the germicidal properties of UV light. The at least one UV light source 6 may include at least one LED, which is configured for emitting UV light.

The UV light emitted by the at least one UV light source 6 may have a wavelength of less than 400 nm, in particular a wavelength in the range of between 150 nm and 300 nm, more particularly a wavelength in the range of between 200 nm and 270 nm.

The system 2 further comprises a light detector 8 and a controller 10.

The light detector 8 is responsive to visible light and to UV light and is configured for providing sensor outputs, representing the detected light, to the controller 10.

The controller 10 is configured for monitoring an operational status of the at least one switchable UV light source, based on sensor outputs received from the light detector 8, and for selectively activating and deactivating the at least one lighting device and the at least one UV light source 6.

The controller 10 is in particular configured for activating the at least one UV light source 6 and for receiving a first sensor output, provided by the light detector 8 while the at least one UV light source 6 is activated. The controller 10 is further configured for deactivating the at least one switchable UV light source 6 and for receiving a second sensor output, which is provided by the light detector 8 while the at least one UV light source 6 is deactivated.

Alternatively, the controller 10 may be configured for receiving a first sensor output, which is provided by the light detector 8 while the at least one UV light source 6 is deactivated; for activating the at least one switchable UV light source 6; and for receiving a second sensor output, which is provided by the light detector 8 while the at least one UV light source 6 is activated.

The controller 10 monitors the operational status of the at least one switchable UV light source 6 by comparing the first and second sensor outputs with each other. The controller 10 is further configured for controlling the at least one switchable UV light source 6 based on said monitoring of the operational status of the at least one switchable UV light source 6.

Details of different modes of operation will be discussed in more detail further below.

Figure 5 shows a schematic cross-sectional view of a light detector 8, as it may be employed in a system 2 according to an exemplary embodiment of the invention.

The light detector 8 includes a photo sensor 80 sensitive to visible light, which is mounted to a support 81, for example to a printed circuit board 81.

The photo sensor 80 may in particular be configured for providing spectral information about the light detected by the photo sensor 80, i.e. the photo sensor 80 may provide information about the intensity of the detected light as a function of the wavelength of the detected light.

The photo sensor 80, for example, may comprise a plurality of detection channels, with the plurality of detection channels having different sensitivities to different ranges of electromagnetic radiation, so that the photo sensor 80 is capable to provide separate intensity information for each range of electromagnetic radiation, respectively.

The photo sensor 80 may, for example, be a multi-color photo sensor, wherein the plurality of detection channels have different sensitivities to different ranges of electromagnetic radiation in the range of visible light, e.g. in the range between 350 nm and 800 nm.

In order to allow the photo sensor 80 to detect UV light, the photo sensor 80 may be at least partially covered with a wavelength converting coating 82, which is configured for emitting visible light when it is excited by UV light.

In the embodiment depicted in Figure 5, the wavelength converting coating 82 includes a translucent adhesive 84, which is applied to an upper surface of the photo sensor 80 on a side opposite to the support 81, and a wavelength converting substance 86, which is applied to a side of the adhesive 84 facing away from the photo sensor 80.

The wavelength converting substance 86 may cover the upper surface of the adhesive 84 facing away from the photo sensor 80 completely. Alternatively, the wavelength converting substance 86 may cover the upper surface of the adhesive 84 only partly, in order to allow visible light to pass through portions of the translucent adhesive 84, which are not covered by the wavelength converting substance 86, in an unimpeded manner.

In an alternative embodiment, which is not explicitly shown in the figures, the light transmissive adhesive 84 may include a thin quartz window that encapsulates the wavelength converting substance 86 underneath.

The wavelength converting substance 86 may include a phosphor material, in particular at least one of Y₂O₃:Eu³⁺, Sr₂AL₆O₁₁:Eu²⁺, BaMgAl₁₀O₁₇:Eu²⁺, and LaPO₄:Ce³⁺,TB³⁺. These phosphor materials have been found as well suited for converting UV light into visible light.

The wavelength of the visible light, into which the UV light is converted, depends on the material used for the wavelength converting substance 86. In other words, the wavelength of the visible light, into which the UV light is converted, may be selected by choosing the material of the wavelength converting substance 86.

Y₂O₃:Eu³⁺, for example, converts UV light very efficiently into visible red light having a wavelength of approximately 611 nm. Sr₂AL₆O₁₁:Eu²⁺ and BaMgAl₁₀O₁₇:Eu²⁺ convert UV light to visible blue light, and LaPO₄:Ce³⁺,TB³⁺ emits visible green light, when it is excited by UV light.

All these materials emit a very narrow spectrum of visible light, i.e. a spectrum of visible light in which at least 90 % of the light is emitted within a range of wavelengths having a width of 50 nm. Providing such a narrow spectrum of visible light may allow for reliably detecting and identifying the converted visible light, which is emitted by the wavelength converting substance 86, when it is excited by UV light.

Alternatively or additionally to a phosphor material, as it has been described before, the wavelength converting substance 86 may include quantum dots for converting the UV light, emitted by the at least one UV light source 6, into visible light, which is detectable by the photo sensor 80.

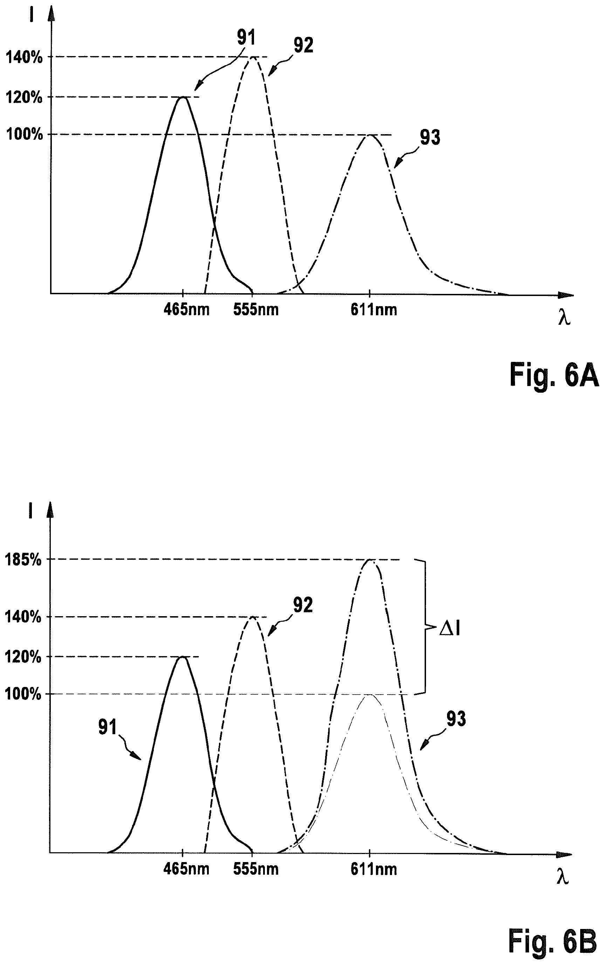

Figures 6A and 6B illustrate examples of light intensities I, plotted on the vertical axis, as detected by the light detector 8 when the at least one UV light source 6 is switched off (Figure 6A) and when the at least one UV light source 6 is switched on (Figure 6B). The light intensities I are depicted as a function of the wavelength λ, which is plotted on the horizontal axis.

Figure 6A depicts the spectral distribution of the light emitted by the at least one lighting device 4 comprising three peaks 91, 92, 93, namely a first peak 91 centered at approximately 465 nm corresponding to blue light, a second peak 92 centered at approximately 555 nm corresponding to green light, and a third peak 93 centered at approximately 611 nm corresponding to red light.

A spectral distribution, as it is depicted in Figure 6A, is detected and stored when the at least one UV light source 6 is deactivated. In addition to light emitted by the at least one lighting device 4 provided within the aircraft 100, the spectral distribution may include further spectral components (not shown), which may result from external light, in particular sun light, which enters through windows into the aircraft 100.

Figure 6B depicts the spectral distribution of the light detected by the light detector 8 when the at least one UV light source 6 is activated and the UV light, emitted by the at least one UV light source 6, is converted into visible light, in particular into red light having a wavelength spectrum centered around 611 nm, by Y₂O₃:Eu³⁺, which has been applied to the surface of the photo sensor 80 as the wavelength converting substance 86.

The comparison of Figures 6A and 6B shows that the first and second peaks 91, 92, corresponding to blue light and green light, do not change when the at least one UV light source 6 is activated. The height of the third peak 93, corresponding to the intensity of detected red light, however, increases considerably when the at least one UV light source 6 is activated. In the examples depicted in Figures 6A and 6B, the height of the third peak 93 almost doubles when the at least one UV light source 6 is activated.

This drastic increase ΔI of the detected intensity of red light allows the controller 10 to reliably confirm that the at least one UV light source 6 is activated. It further allows the controller 10 to determine an indication regarding the intensity of the UV light, emitted by the at least one UV light source 6, from the change of the height of the third peak 93.

In case a different material than Y₂O₃:Eu³⁺ is employed as the wavelength converting substance 86 for converting the UV light, emitted by the least one UV light source 6, into visible light, the first peak 91 and/or the second peak 92, corresponding to blue light and green light, respectively, may increase instead of or in addition to the third peak 93.

In particular, if Sr₂AL₆O₁₁:Eu²⁺ or BaMgAl₁₀O₁₇:Eu²⁺ are included in the wavelength converting substance 86, the first peak 91 corresponding to blue light will increase, and the second peak 92 corresponding to green light will increase if LaPO₄:Ce³⁺,TB³⁺ is included in the wavelength converting substance 86.

By comparing a first sensor output, provided by the light detector 8 when the at least one switchable UV light source 6 is activated, as it its depicted in Figure 6B, with a second sensor output, which is provided by the light detector 8 when the at least one switchable UV light source 6 is deactivated, as it is depicted in Figure 6A, the controller 10 is capable to reliably determine whether the at least one switchable UV light source 6 has been activated. This may be true even under varying ambient light conditions. The ambient light conditions may, for example, change due to a change of external light, in particular sun light, shining into the aircraft 100.

The controller 10 is further capable to determine an indication regarding the intensity of the UV light and to control the operation of the at least one UV light source 6 based on the information about the intensity of the UV light, provided by the light detector 8.

Controlling the at least one switchable UV light source 6 may include adjusting the intensity of UV light, emitted by the at least one switchable UV light source 6, by controlling the operation of the at least one UV light source 6. The intensity of UV light, emitted by the at least one UV light source 6, may for example be adjusted so that the intensity of the UV light exceeds a predefined minimum intensity threshold and/or so that the intensity does not exceed a predefined maximum intensity threshold.

Controlling the at least one switchable UV light source 6 may also include adjusting a period of time, for which the at least one switchable UV light source 6 is activated. The period of time, for which the at least one switchable UV light source 6 is activated, may in particular be changed as a function of the detected intensity of UV light.

For example, the period of time, for which the at least one switchable UV light source 6 is activated, may be extended when the detected UV intensity is low, and the period of time, for which the at least one switchable UV light source 6 is activated, may be shortened when the detected UV intensity is high. In this way, a substantially constant disinfection effect may be achieved, independent of potential changes in the intensity of the UV light, which may occur for example due to aging of the at least one UV light source 6.

The controller 10 may, for example, be configured for calculating an indication regarding a total amount of UV light, which is emitted by the at least one switchable UV light source 6 over time, by accumulating the sensor outputs over time. The controller 10 may further be configured for providing a confirmation signal, when the indication exceeds a predefined threshold. The confirmation signal may include a visual and/or an acoustic signal. The confirmation signal may also include a control signal, which causes deactivating the at least one switchable UV light source. Such a configuration may allow for indicating that an amount of UV light, which is considered sufficient for reliably disinfecting at least one surface by illumination with UV light, has been detected. It may further allow for automatically deactivating the at least one switchable UV light source after a sufficient amount of UV light has been detected. This may allow for a reliable automatic disinfection of surfaces within the aircraft 100.

Optionally, the confirmation signal may further include a signal which is transmitted to a board computer of the aircraft 100 and/or a log signal, which allows for logging the operation of the at least one UV light source 6. Logging the operation of the at least one UV light source 6 may allow for documenting that surfaces within the aircraft 100 have been disinfected regularly and sufficiently.

Logging the operation of the at least one UV light source 6 may further allow for monitoring the periods of operational time of the at least one UV light source 6 needed for sufficient disinfection. An increase of the periods of operational time needed for sufficient disinfection may indicate an aging of the at least one UV light source 6. In consequence, an aged UV light source 6 may be replaced in good time before it completely fails or becomes too weak for allowing a reliable disinfection.

It is intended that the invention not be limited to the particular embodiment disclosed, but that the invention will include all embodiments falling within the scope of the appended claims.

## Claims

1. System (2) for providing and monitoring UV illumination in an interior of an aircraft (100), wherein the system (2) comprises:
- at least one switchable UV light source (6);
- a light detector (8), responsive to visible light and UV light and configured for providing sensor outputs regarding detected light;
- a controller (10) for monitoring an operational status of the at least one switchable UV light source (6), wherein the controller (10) is configured to determine the operational status of the at least one switchable UV light source (6) by comparing a first sensor output, provided by the light detector (8) when the at least one switchable UV light source (6) is activated, with a second sensor output, provided by the light detector (8) when the at least one switchable UV light source (6) is deactivated.

2. System (2) according to claim 1, wherein the light detector (8) includes a photo sensor (80), which is sensitive to visible light, and a wavelength converting coating (82), with the wavelength converting coating (82) emitting visible light when being excited by UV light.

3. System (2) according to claim 2, wherein the photo sensor (80) comprises a plurality of detection channels, with the plurality of detection channels having different sensitivities to different ranges of electromagnetic radiation, wherein the photo sensor (80) is in particular a multi-color photo sensor (80), with the plurality of detection channels having different sensitivities to different ranges of electromagnetic radiation in the range of visible light.

4. System (2) according to claim 2 or 3, wherein the wavelength converting coating (82) comprises a light transmissive adhesive (84), in particular a translucent adhesive (84), and wherein a wavelength converting substance (86) is adhered to the photo sensor (80) via the light transmissive adhesive (84).

5. System (2) according to claim 4, wherein the wavelength converting substance (86) includes a phosphor material, in particular at least one of Y₂O₃:Eu³⁺, Sr₂AL₆O₁₁:Eu²⁺, BaMgAl₁₀O₁₇:Eu²⁺, and LaPO₄:Ce³⁺,TB³⁺.

6. System (2) according to claim 4 or 5, wherein the wavelength converting substance (86), when excited with UV light, emits visible light in the range of between 600 nm and 650 nm, in particular in the range of between 605 nm and 615 nm.

7. System (2) according to any of the preceding claims, wherein the the light detector (8) includes quantum dots.

8. System (2) according to any of the preceding claims, wherein the system (2) is configured for controlling the at least one switchable UV light source (6) based on said monitoring of the operational status of the at least one switchable UV light source (6); wherein controlling the at least one switchable UV light source (6) in particular includes adjusting an intensity of UV light, emitted by the at least one switchable UV light source (6), and/or adjusting a period of time, for which the at least one switchable UV light source (6) is activated.

9. System (2) according to any of the preceding claims, wherein the system (2) is configured for calculating an indication regarding a total amount of UV light, emitted by the at least one switchable UV light source (6) over time, by accumulating the sensor outputs over time, and wherein the system (2) is configured for providing a confirmation signal, when the indication regarding the total amount of UV light exceeds a predefined threshold; wherein the confirmation signal is in particular a visual confirmation signal and/or an acoustic confirmation signal, and/or wherein the system (2) is configured for deactivating the at least one switchable UV light source (6), when the confirmation signal is issued.

10. Aircraft (100) comprising at least one system (2) for providing and monitoring UV illumination according to any of the preceding claims, wherein the at least one system (2) is located in at least one of a cockpit (103) and a passenger cabin (104) of the aircraft (100), wherein the at least one system (2) is in particular located in a lavatory (108-108d) and/or in a galley (110) provided within the passenger cabin (104) of the aircraft (100).

11. Method of providing and monitoring UV illumination in an interior of an aircraft (100), wherein the method includes:
- detecting light within the interior of the aircraft (100) with a light detector (8), while at least one switchable UV light source (6) is deactivated, and providing a first sensor output, which is a function of the detected light;
- activating the at least one switchable UV light source (6);
- detecting light within the interior of the aircraft (100) with the light detector (8), while the at least one switchable UV light source (6) is activated, and providing a second sensor output, which is a function of the detected light;
- monitoring an operational status of the at least one switchable UV light source (6), wherein monitoring an operational status of the at least one switchable UV light source (6) includes comparing the first sensor output with the second sensor output.

12. Method according to claim 11, wherein the light is detected by a light detector (8) comprising at least one photo sensor (80), which is sensitive to visible light, and a wavelength converting coating (82), which emits visible light when it is excited by UV light.

13. Method according to claim 11 or 12, wherein the method includes controlling the at least one switchable UV light source (6) based on said monitoring of the operation status of the at least one switchable UV light source (6); wherein controlling the at least one switchable UV light source (6) in particular includes adjusting an intensity of light, emitted by the at least one switchable UV light source (6), and/or adjusting a period of time, for which the at least one switchable UV light source (6) is activated.

14. Method according to any of claims 11 to 13, wherein the method includes calculating an indication regarding a total amount of UV light, emitted by the at least one switchable UV light source (6), by accumulating the sensor outputs over time and providing a confirmation signal, when the calculated indication exceeds a predefined threshold; wherein the confirmation signal is in particular a visual confirmation signal and/or an acoustic confirmation signal, and/or wherein the method includes deactivating the at least one switchable UV light source (6), when the confirmation signal has been issued.

15. Method according to any of claims 11 to 14, wherein the method includes illuminating portions of a cockpit (103) or of a passenger cabin (104) of an aircraft (100) with the UV light emitted by the at least one switchable UV light source (6), wherein the method in particular includes illuminating portions of at least one of a lavatory (108a-108d), a galley (110), and a passenger service unit (102), located within the passenger cabin (104), with the UV light.

## Patentansprüche

1. System (2) zum Bereitstellen und Überwachen einer UV-Beleuchtung in einem Innenraum eines Luftfahrzeugs (100), wobei das System (2) Folgendes umfasst:
- mindestens eine schaltbare UV-Lichtquelle (6);
- einen Lichtdetektor (8), der auf sichtbares Licht und UV-Licht reagiert und zum Bereitstellen von Sensorausgaben bezüglich des erfassten Lichts konfiguriert ist;
- eine Steuerung (10) zum Überwachen eines Betriebsstatus der mindestens einen schaltbaren UV-Lichtquelle (6), wobei die Steuerung (10) dazu konfiguriert ist, den Betriebsstatus der mindestens einen schaltbaren UV-Lichtquelle (6) durch Vergleichen einer ersten Sensorausgabe, die durch den Lichtdetektor (8) bereitgestellt wird, wenn die mindestens eine schaltbare UV-Lichtquelle (6) aktiviert ist, mit einer zweiten Sensorausgabe, die durch den Lichtdetektor (8) bereitgestellt wird, wenn die mindestens eine schaltbare UV-Lichtquelle (6) deaktiviert ist, zu bestimmen.

2. System (2) nach Anspruch 1, wobei der Lichtdetektor (8) einen gegenüber sichtbarem Licht empfindlichen Fotosensor (80) und eine wellenlängenkonvertierende Beschichtung (82) beinhaltet, wobei die wellenlängenkonvertierende Beschichtung (82) bei Anregung durch UV-Licht sichtbares Licht emittiert.

3. System (2) nach Anspruch 2, wobei der Fotosensor (80) eine Mehrzahl von Erfassungskanälen umfasst, wobei die Mehrzahl von Erfassungskanälen unterschiedliche Empfindlichkeiten gegenüber unterschiedlichen Bereichen elektromagnetischer Strahlung aufweist, wobei der Fotosensor (80) insbesondere ein Mehrfarben-Fotosensor (80) ist, wobei die Mehrzahl von Erfassungskanälen unterschiedliche Empfindlichkeiten gegenüber unterschiedlichen Bereichen elektromagnetischer Strahlung im Bereich des sichtbaren Lichts aufweist.

4. System (2) nach Anspruch 2 oder 3, wobei die wellenlängenkonvertierende Beschichtung (82) einen lichtdurchlässigen Klebstoff (84), insbesondere einen transluzenten Klebstoff (84), umfasst, und wobei eine wellenlängenkonvertierende Substanz (86) mit dem lichtdurchlässigen Klebstoff (84) auf den Fotosensor (80) geklebt ist.

5. System (2) nach Anspruch 4, wobei die wellenlängenkonvertierende Substanz (86) ein Phosphormaterial, insbesondere mindestens eines von Y₂O₃:Eu³⁺, Sr₂AL₆O₁₁:Eu²⁺, BaMgAI₁₀O₁₇:Eu²⁺ und LaPO₄:Ce³⁺, TB³⁺, beinhaltet.

6. System (2) nach Anspruch 4 oder 5, wobei die wellenlängenkonvertierende Substanz (86) bei Anregung mit UV-Licht sichtbares Licht im Bereich zwischen 600 nm und 650 nm, insbesondere im Bereich zwischen 605 nm und 615 nm, emittiert.

7. System (2) nach einem der vorhergehenden Ansprüche, wobei der Lichtdetektor (8) Quantenpunkte beinhaltet.

8. System (2) nach einem der vorhergehenden Ansprüche, wobei das System (2) zum Steuern der mindestens einen schaltbaren UV-Lichtquelle (6) basierend auf der Überwachung des Betriebsstatus der mindestens einen schaltbaren UV-Lichtquelle (6) konfiguriert ist; wobei das Steuern der mindestens einen schaltbaren UV-Lichtquelle (6) insbesondere ein Anpassen einer Intensität von UV-Licht, das durch die mindestens eine schaltbare UV-Lichtquelle (6) emittiert wird, und/oder ein Anpassen einer Zeitdauer, für die die mindestens eine schaltbare UV-Lichtquelle (6) aktiviert ist, beinhaltet.

9. System (2) nach einem der vorhergehenden Ansprüche, wobei das System (2) zum Berechnen einer Angabe bezüglich einer Gesamtmenge an UV-Licht, die durch die mindestens eine schaltbare UV-Lichtquelle (6) über die Zeit emittiert wird, durch Akkumulieren der Sensorausgaben über die Zeit konfiguriert ist und wobei das System (2) zum Bereitstellen eines Bestätigungssignals konfiguriert ist, wenn die Angabe bezüglich der Gesamtmenge an UV-Licht einen vordefinierten Schwellenwert überschreitet; wobei das Bestätigungssignal insbesondere ein visuelles Bestätigungssignal und/oder ein akustisches Bestätigungssignal ist und/oder wobei das System (2) zum Deaktivieren der mindestens einen schaltbaren UV-Lichtquelle (6) konfiguriert ist, wenn das Bestätigungssignal ausgegeben wird.

10. Luftfahrzeug (100), umfassend mindestens ein System (2) zum Bereitstellen und Überwachen einer UV-Beleuchtung nach einem der vorhergehenden Ansprüche, wobei sich das mindestens eine System (2) in mindestens einem von einem Cockpit (103) und einer Passagierkabine (104) des Luftfahrzeugs (100) befindet, wobei sich das mindestens eine System (2) insbesondere in einer Toilette (108-108d) und/oder in einer Bordküche (110) befindet, die innerhalb der Passagierkabine (104) des Luftfahrzeugs (100) bereitgestellt ist.

11. Verfahren zum Bereitstellen und Überwachen einer UV-Beleuchtung in einem Inneren eines Luftfahrzeugs (100), wobei das Verfahren Folgendes beinhaltet:
- Erfassen von Licht im Inneren des Luftfahrzeugs (100) mit einem Lichtdetektor (8), während mindestens eine schaltbare UV-Lichtquelle (6) deaktiviert ist, und Bereitstellen einer ersten Sensorausgabe, die von dem erfassten Licht abhängig ist;
- Aktivieren der mindestens einen schaltbaren UV-Lichtquelle (6) ;
- Erfassen von Licht im Inneren des Luftfahrzeugs (100) mit dem Lichtdetektor (8), während die mindestens eine schaltbare UV-Lichtquelle (6) aktiviert ist, und Bereitstellen einer zweiten Sensorausgabe, die von dem erfassten Licht abhängig ist;
- Überwachen eines Betriebsstatus der mindestens einen schaltbaren UV-Lichtquelle (6), wobei das Überwachen eines Betriebsstatus der mindestens einen schaltbaren UV-Lichtquelle (6) ein Vergleichen der ersten Sensorausgabe mit der zweiten Sensorausgabe beinhaltet.

12. Verfahren nach Anspruch 11, wobei das Licht durch einen Lichtdetektor (8) erfasst wird, der mindestens einen gegenüber sichtbarem Licht empfindlichen Fotosensor (80) und eine wellenlängenkonvertierende Beschichtung (82), die bei Anregung durch UV-Licht sichtbares Licht emittiert, umfasst.

13. Verfahren nach Anspruch 11 oder 12, wobei das Verfahren das Steuern der mindestens einen schaltbaren UV-Lichtquelle (6) basierend auf dem Überwachen des Betriebsstatus der mindestens einen schaltbaren UV-Lichtquelle (6) beinhaltet; wobei das Steuern der mindestens einen schaltbaren UV-Lichtquelle (6) insbesondere ein Anpassen einer Intensität von Licht, das durch die mindestens eine schaltbare UV-Lichtquelle (6) emittiert wird, und/oder ein Anpassen einer Zeitdauer, für die die mindestens eine schaltbare UV-Lichtquelle (6) aktiviert ist, beinhaltet.

14. Verfahren nach einem der Ansprüche 11 bis 13, wobei das Verfahren ein Berechnen einer Angabe bezüglich einer Gesamtmenge an UV-Licht, die durch die mindestens eine schaltbare UV-Lichtquelle (6) emittiert wird, durch Akkumulieren der Sensorausgaben über die Zeit, und ein Bereitstellen eines Bestätigungssignals, wenn die berechnete Angabe einen vordefinierten Schwellenwert überschreitet, beinhaltet; wobei das Bestätigungssignal insbesondere ein visuelles Bestätigungssignal und/oder ein akustisches Bestätigungssignal ist und/oder wobei das Verfahren ein Deaktivieren der mindestens einen schaltbaren UV-Lichtquelle (6) beinhaltet, wenn das Bestätigungssignal ausgegeben wurde.

15. Verfahren nach einem der Ansprüche 11 bis 14, wobei das Verfahren ein Beleuchten von Teilen eines Cockpits (103) oder einer Passagierkabine (104) eines Luftfahrzeugs (100) mit dem durch die mindestens eine schaltbare UV-Lichtquelle (6) emittierten UV-Licht beinhaltet, wobei das Verfahren insbesondere ein Beleuchten von Teilen von mindestens einem von einer Toilette (108a-108d), einer Bordküche (110) und einer Passagierserviceeinheit (102), die sich innerhalb der Passagierkabine (104) befinden, mit dem UV-Licht beinhaltet.

## Revendications

1. Système (2) de fourniture et de surveillance d'un éclairage UV dans un intérieur d'un aéronef (100), dans lequel le système (2) comprend:
- au moins une source de lumière UV commutable (6);
- un détecteur de lumière (8), sensible à la lumière visible et à la lumière UV et configuré pour fournir des sorties de capteur concernant la lumière détectée;
- un contrôleur (10) pour surveiller un état opérationnel de l'au moins une source de lumière UV commutable (6), dans lequel le contrôleur (10) est configuré pour déterminer l'état opérationnel de l'au moins une source de lumière UV commutable (6) en comparant une première sortie de capteur, fournie par le détecteur de lumière (8) lorsque l'au moins une source de lumière UV commutable (6) est activée, avec une seconde sortie de capteur, fournie par le détecteur de lumière (8) lorsque l'au moins une source de lumière UV commutable (6) est désactivée.

2. Système (2) selon la revendication 1, dans lequel le détecteur de lumière (8) comprend un photocapteur (80), qui est sensible à la lumière visible, et un revêtement de conversion de longueur d'onde (82), le revêtement de conversion de longueur d'onde (82) émettant de la lumière visible lorsqu'il est excité par une lumière UV.

3. Système (2) selon la revendication 2, dans lequel le photocapteur (80) comprend une pluralité de canaux de détection, la pluralité de canaux de détection ayant des sensibilités différentes à différentes plages de rayonnement électromagnétique, dans lequel le photocapteur (80) est en particulier, un photocapteur multicolore (80), la pluralité de canaux de détection ayant différentes sensibilités à différentes plages de rayonnement électromagnétique dans la plage de la lumière visible.

4. Système (2) selon la revendication 2 ou 3, dans lequel le revêtement de conversion de longueur d'onde (82) comprend un adhésif transmettant la lumière (84), en particulier un adhésif translucide (84), et dans lequel une substance de conversion de longueur d'onde (86) est collée au photocapteur (80) via l'adhésif transmettant la lumière (84).

5. Système (2) selon la revendication 4, dans lequel la substance de conversion de longueur d'onde (86) comprend un matériau phosphore, en particulier au moins l'un parmi Y₂O₃:Eu³⁺, Sr₂ALO₁₁:Eu²⁺, BaMgAl₁₀O₁₇:Eu²⁺ et LaPO₄:Ce³⁺, TB³⁺.

6. Système (2) selon la revendication 4 ou 5, dans lequel la substance de conversion de longueur d'onde (86), lorsqu'elle est excitée par de la lumière UV, émet de la lumière visible dans la plage comprise entre 600 nm et 650 nm, en particulier dans la plage comprise entre 605 nm et 615 nm.

7. Système (2) selon l'une quelconque des revendications précédentes, dans lequel le détecteur de lumière (8) comprend des points quantiques.

8. Système (2) selon l'une quelconque des revendications précédentes, dans lequel le système (2) est configuré pour contrôler l'au moins une source de lumière UV commutable (6) sur la base de ladite surveillance de l'état opérationnel de l'au moins une source de lumière UV commutable (6); dans lequel le contrôle de l'au moins une source de lumière UV commutable (6) comprend en particulier le réglage d'une intensité de lumière UV, émise par l'au moins une source de lumière UV commutable (6), et/ou le réglage d'une période de temps pendant laquelle au moins une source de lumière UV commutable (6) est activée.

9. Système (2) selon l'une quelconque des revendications précédentes, dans lequel le système (2) est configuré pour calculer une indication concernant une quantité totale de lumière UV, émise par l'au moins une source de lumière UV commutable (6) au fil du temps, en accumulant les sorties de capteur au fil du temps, et dans lequel le système (2) est configuré pour fournir un signal de confirmation, lorsque l'indication concernant la quantité totale de lumière UV dépasse un seuil prédéfini; dans lequel le signal de confirmation est en particulier un signal de confirmation visuel et/ou un signal de confirmation acoustique, et/ou dans lequel le système (2) est configuré pour désactiver l'au moins une source de lumière UV commutable (6) lorsque le signal de confirmation est émis.

10. Aéronef (100) comprenant au moins un système (2) pour fournir et surveiller un éclairage UV selon l'une quelconque des revendications précédentes, dans lequel l'au moins un système (2) est situé dans au moins l'un d'un cockpit (103) et d'une cabine passagers (104) de l'aéronef (100), dans lequel l'au moins un système (2) est en particulier situé dans des toilettes (108-108d) et/ou dans une cuisine (110) prévue à l'intérieur de la cabine passagers (104) de l'aéronef (100).

11. Procédé de fourniture et de surveillance d'un éclairage UV dans un intérieur d'un aéronef (100), dans lequel le procédé comprend:
- la détection d'une lumière à l'intérieur de l'aéronef (100) avec un détecteur de lumière (8), tandis qu'au moins une source de lumière UV commutable (6) est désactivée, et la fourniture d'une première sortie de capteur, qui est une fonction de la lumière détectée;
- l'activation de l'au moins une source de lumière UV commutable (6) ;
- la détection d'une lumière à l'intérieur de l'aéronef (100) avec un détecteur de lumière (8), tandis que l'au moins une source de lumière UV commutable (6) est activée, et la fourniture d'une seconde sortie de capteur, qui est une fonction de la lumière détectée;
- la surveillance d'un état opérationnel de l'au moins une source de lumière UV commutable (6), dans lequel la surveillance d'un état opérationnel de l'au moins une source de lumière UV commutable (6) comprend la comparaison de la première sortie de capteur avec la seconde sortie de capteur.

12. Procédé selon la revendication 11, dans lequel la lumière est détectée par un détecteur de lumière (8) comprenant au moins un photodétecteur (80), qui est sensible à la lumière visible, et un revêtement de conversion de longueur d'onde (82), qui émet de la lumière visible lorsqu'il est excité par de la lumière UV.

13. Procédé selon la revendication 11 ou 12, dans lequel le procédé comprend le contrôle de l'au moins une source de lumière UV commutable (6) sur la base de ladite surveillance de l'état opérationnel de l'au moins une source de lumière UV commutable (6); dans lequel le contrôle de l'au moins une source de lumière UV commutable (6) comprend en particulier le réglage d'une intensité de lumière UV, émise par l'au moins une source de lumière UV commutable (6), et/ou le réglage d'une période de temps pendant laquelle au moins une source de lumière UV commutable (6) est activée.

14. Procédé selon l'une quelconque des revendications 11 à 13, dans lequel le procédé comprend le calcul d'une indication concernant une quantité totale de lumière UV, émise par l'au moins une source de lumière UV commutable (6), en accumulant les sorties de capteur au fil du temps et en fournissant un signal de confirmation, lorsque l'indication calculée dépasse un seuil prédéfini; dans lequel le signal de confirmation est en particulier un signal de confirmation visuel et/ou un signal de confirmation acoustique, et/ou dans lequel le procédé comprend la désactivation de l'au moins une source de lumière UV commutable (6), lorsque le signal de confirmation a été émis.

15. Procédé selon l'une quelconque des revendications 11 à 14, dans lequel le procédé comprend l'éclairage de parties d'un cockpit (103) ou d'une cabine passagers (104) d'un aéronef (100) avec la lumière UV émise par l'au moins une source de lumière UV commutable (6), dans lequel le procédé comprend en particulier l'éclairage de parties d'au moins l'une des toilettes (108a-108d), d'une cuisine (110) et d'une unité de service aux passagers (102), située à l'intérieur de la cabine de passagers (104), avec la lumière UV.
